# EUROPÄISCHE PATENTSCHRIFT

(11) **EP 1 328 573 B1**
(45) Veröffentlichungstag und Bekanntmachung des Hinweises auf die Patenterteilung: **09.11.2005**
(21) Anmeldenummer: 01985717.6
(22) Anmeldetag: 20.09.2001
(51) Int. Cl.: C08J 9/00, C08J 9/14, C08J 9/36, A61L 15/42

(54) **HYDROPHILE, OFFENZELLIGE, ELASTISCHE SCHAUMSTOFFE AUF BASIS VON MELAMIN/FORMALDEHYD-HARZEN, IHRE HERSTELLUNG UND IHRE VERWENDUNG IN HYGIENEARTIKELN**
HYDROPHILIC, OPEN-CELL, ELASTIC FOAMS WITH A MELAMINE/FORMALDEHYDE RESIN BASE, PRODUCTION THEREOF AND USE THEREOF IN HYGIENE PRODUCTS
PRODUIT ALVEOLAIRE ELASTIQUE, HYDROPHILE ET A ALVEOLES OUVERTES A BASE DE RESINES DE MELAMINE/FORMALDEHYDE, LEUR PRODUCTION ET LEUR UTILISATION DANS DES ARTICLES D'HYGIENE

(30) Priorität: 27.09.2000 DE 10047717
(43) Veröffentlichungstag der Anmeldung: 23.07.2003
(73) Patentinhaber: BASF Aktiengesellschaft, 67056 Ludwigshafen (DE)
(72) Erfinder: HÄHNLE, Hans-Joachim, 67435 Neustadt (DE); BAUMGARTL, Horst, 55122 Mainz (DE); BECK, Martin, 67133 Maxdorf (DE); HERFERT, Norbert, 63674 Altenstadt (DE); MOHR, Bernhard, 74523 Schwäbisch Hall (DE); HUFF, Jürgen, 67063 Ludwigshafen (DE)
(86) Internationale Anmeldenummer: PCT/EP2001/010848
(87) Internationale Veröffentlichungsnummer: WO 2002/026871

(56) Entgegenhaltungen:
- EP-A- 0 017 671
- EP-A- 0 220 506
- DE-A- 3 138 862
- US-A- 5 292 777

## Beschreibung

Die Erfindung betrifft hydrophile, offenzellige, elastische Schaumstoffe auf Basis von Melamin/Formaldehyd-Harzen, ihre Herstellung und ihre Verwendung in Hygieneartikeln.

Aus der EP-A-0 017 621 und EP-A-0 017 672 sind offenzellige, elastische Schaumstoffe auf Basis von Melamin/Formaldehyd-Kondensationsprodukten sowie Verfahren zu ihrer Herstellung bekannt. Nach dem aus der EP-A-0 037 470 bekannten Verfahren erfolgt die Herstellung von offenzelligen, elastischen Schaumstoffen aus Melamin/Formaldehyd-Kondensationsprodukten besonders vorteilhaft durch Einwirkung von Mikrowellenenergie (Ultrahochfrequenzbestrahlung) auf eine wäßrige Lösung oder Dispersion, die jeweils ein Melamin/Formaldehyd-Vorkondensat, einen Emulgator, ein Treibmittel und einen Härter enthalten. Die Lösung bzw. Dispersion wird dabei derart erhitzt, daß sie aufschäumt und das Vorkondensat aushärtet. Die so erhältlichen Schaumstoffe emittieren geringe Mengen an Formaldehyd, wobei die Formaldehyd-Emission mit zunehmender Temperatur und Feuchte des Schaumstoffs ansteigt.

Der Aufbau von Hygieneartikeln und die Verwendung von offenzelligen Schaumstoffen aus Melamin/Formaldehyd-Harzen als absorbierende Zwischenschicht werden in der älteren, nicht vorveröffentlichten DE-Anmeldung Nr. 100 34 505.0 ausführlich beschrieben.
Die dort angeführten Schaumstoffe aus Melamin/Formaldehyd-Harzen sind zwar hochgradig hydrophil, weisen aber eine verhältnismäßig hohe Formaldehydabgabe bei Kontakt mit Körperflüssigkeiten auf. Dadurch werden die Einsatzmöglichkeiten derartiger Schaumstoffe in Hygieneartikeln erheblich eingeschränkt.

Aus der älteren, nicht vorveröffentlichten DE-Anmeldung Nr. 100 27 770.5 ist die Herstellung von Schaumstoffen aus formaldehydarmen, offenzelligen Melamin/Formaldehyd-Harzen mit einem Molverhältnis von Melamin/Formaldehyd von 1 : 1,0 bis 1 : 1,9 beschrieben. Diese Schaumstoffe emittieren selbst unter den im Hygienebereich üblichen warm / feucht Bedingungen weniger als 30 mg Formaldehyd/kg Schaumstoff (EU-Norm EN ISO 14 184-1, Wasserlagerung 1 h bei 40 Grad C). Sie erfüllen damit die an Babybekleidung gestellten Forderungen des ÖKOTEX-Standard 100 (Qualitätssiegel der Textilindustrie für besonders schadstoffarme Textilien). Die erhebliche Reduktion der Formaldehyd-Emission wird jedoch mit einem partiellen Verlust der hydrophilen Eigenschaften des Schaumstoffes erkauft, wodurch die Flüssigkeitsaufnahmegeschwindigkeit solcher Schaumstoffschichten absinkt.

Aus der WO-A-96/21682 sind Schaumstoffe bekannt, die aufgrund ihrer offenzelligen Struktur hervorragend zur Absorption wäßriger Körperflüssigkeiten, insbesondere zur Blutabsorption geeignet sind. Die Schaumstoffe werden erhalten durch Polymerisation von (C₄-C₁₄)-Alkylacrylaten, (C₆-C₁₆)-Alkylmethacrylaten, (C₄-C₁₂)-Alkylstyrolen als Monomere, bevorzugt Styrol und Ethylstyrol als Comonomere, des weiteren aromatische Polyvinylverbindungen als Vernetzer; optional polyfunktionelle Acrylate, Methacrylate, Acrylamide und Methacrylamide und Mischungen davon als zusätzliche Vernetzersubstanzen. Die Polymerisation findet innerhalb einer High-Internal-Phase-Emulsion (HIPE) vom Typ W/O statt, wobei das Gewichtsverhältnis von Wasserphase zu Ölphase 20 :1 bis 125 : 1 beträgt. Nach beendeter Polymerisation erfolgt das Waschen' und Trocknen der Polymerschäume.

WO-A-97/07832, US-A-5,318,554 und US-A-5,550,167 betreffen die Herstellung offenzelliger Schaumstoffe auf Basis von HIPE-Emulsionen und deren Verwendung zur Absorption wäßriger Körperflüssigkeiten. Die offenzelligen Schäume werden allerdings immer zusammen mit anderen Komponenten eingesetzt, die im Hygieneartikel die endgültige Absorption (Immobilisierung) der Körperflüssigkeiten übernehmen. Diese Materialien haben zwar gute anwendungstechnische Eigenschaften, aber weisen dennoch klare Nachteile auf. So ist ihre Herstellung extrem aufwendig, weil der Prozess verfahrenstechnisch nur schwer zu kontrollieren ist. Der enorme Aufwand an wässriger Phase (wässrige Salzlösung) ist weder ökonomisch noch ökologisch sinnvoll. Die Materialien tragen außerdem zur Hydrophilierung an der Oberfläche eine Salzschicht. Diese Schicht kann während des Gebrauchs abgelöst und in das Speichermedium des absorbent core ausgewaschen werden. Als Speichermedium werden in der Regel Superabsorbern eingesetzt. Es ist bekannt, dass Superabsorber das Phänomen der "Salzvergiftung" zeigen, d.h. ihre Aufnahmekapazität geht mit steigendem Salzgehalt der zu absorbierenden Lösung dramatisch zurück. Daher ist es nachteilig, die Salzfracht in den zu absorbierenden Körperflüssigkeiten zusätzlich zu erhöhen.

Der vorliegenden Erfindung liegt daher die Aufgabe zugrunde, offenzellige, elastische Schaumstoffe auf Basis von Melamin/Formaldehydharzen zur Verfügung zu stellen, die hydrophil sind und deren Formaldehyd-Emission gegenüber bekannten Schaumstoffen aus Melamin/Formaldehydharzen deutlich erniedrigt ist.

Die Aufgabe wird erfindungsgemäß gelöst mit hydrophilen, offenzelligen, elastischen Schaumstoffen aus Melamin/Formaldehyd-Harzen, die eine Tropfenaufnahmegeschwindigkeit von weniger als 5 Sekunden haben und deren Formaldehyd-Emission nach der EU Norm EN ISO 14184-1 weniger als 100 mg Formaldehyd / kg Schaustoff beträgt.

Solche Schaumstoffe haben beispielsweise eine Dichte von 5 bis 200 g/l, eine spezifische Oberfläche (bestimmt nach BET) von mehr als 0,5 m²/g und eine Free Swell Capacity von mehr als 20 g/g. Sie weisen z.B. im nassen Zustand eine Zugfestigkeit von >60 J/m² auf.

Gegenstand der Erfindung ist außerdem ein Verfahren zur Herstellung von hydrophilen, offenzelligen, elastischen Schaumstoffen, wobei man
(a) eine wäßrige Lösung oder Dispersion, die jeweils mindestens ein Melamin/Formaldehyd-Vorkondensat, einen Emulgator, ein Treibmittel und einen Härter enthalten, unter Ausbildung eines Schaumstoffs und Vernetzen des Vorkondensates erhitzt,
(b) den Schaumstoff dann während einer Zeit von 1 bis 180 Minuten bei 120 bis 300 °C tempert, wobei flüchtige Anteile entfernt werden, und
(c) ihn während des Temperns oder danach mit mindestens einem Hydrophilierungsmittel und/oder mit Ozon, einer Coronaentladung oder einem-Plasma behandelt.

Der Verfahrensschritt (a) ist aus dem Stand der Technik bekannt, vgl. die oben bereits abgehandelten Literaturstellen EP-A-0 017 621, EP-A-0 017 672 und EP-A-0 037 470. Das Molverhältnis von Melamin zu Formaldehyd beträgt beispielsweise 1 : 1,0 bis 1 : 5 und liegt vorzugsweise in dem Bereich von 1 : 1,0 bis 1 : 1,9. Der bevorzugte Bereich ist aus der älteren, nicht vorveröffentlichten DE-Anmeldung Nr. 100 27 770.5 bekannt. Besonders vorteilhaft für die Herstellung formaldehydarmer Melamin/Formaldehyd-Harze ist ein Molverhältnis von Melamin zu Formaldehyd von 1 : 1,3 bis 1 : 1,8. Das Verschäumen gemäß dem Verfahrensschritt (a) geschieht durch Erhitzen der Mischung auf eine Temperatur oberhalb des Siedepunktes des Treibmittels und wird beispielsweise so durchgeführt wird, daß zunächst ein geringer Viskositätsanstieg erfolgt, und der Vernetzungsvorgang unter starker Viskositätserhöhung im wesentlichen erst dann einsetzt, wenn der Schäumvorgang beendet ist. Schäumen der Mischung und Vernetzen des Vorkondensats können jedoch auch gleichzeitig erfolgen. Das Erhitzen der Mischung wird beispielsweise durch Einwirkung von Heißluft, Wasserdampf und/oder Ausnutzen von Reaktionswärme vorgenommen. Das Aufschäumen der wäßrigen Mischung aus Melamin/Formaldehyd-Vorkondensat, Emulgator, Treibmittel und Härter geschieht vorzugsweise durch Einwirkung von Mikrowellenenergie nach dem aus der EP-A-0 0 37 470 bekannten Verfahren.

Struktur und mechanische Eigenschaften der Schaumstoffe sind aus der EP-A- 0 017 672 bekannt:
- Rohdichte nach DIN 53 420 liegt zwischen 1,6 und 30, vorzugsweise zwischen 2 und 20 [g/l]
- Wärmeleitzahl nach DIN 52 612 ist kleiner als 0,06, vorzugsweise kleiner als 0,04 [W.m⁻¹K⁻¹];
- Stauchhärte nach DIN 53 577 bei 60% Stauchung, dividiert durch die Rohdichte liegt unter 0,3, vorzugsweise unter 0,2 [N.cm⁻²/g.l⁻¹], wobei bei der Bestimmung der Stauchhärte bei 60% Stauchung eine Wiedererholung des Schaumstoffs auf 70%, vorzugsweise mindestens 90% und insbesondere 95% seiner ursprünglichen Abmessungen erfolgen muß.
- Elastizitätsmodul in Anlehnung an DIN 53 423, dividiert durch die Rohdichte liegt unter 0,25, vorzugsweise unter 0,15 [N.mm⁻²/g.l⁻¹].
- Biegeweg beim Bruch nach DIN 53 423 ist größer als 10, vorzugsweise größer als 15 [mm];
- Druckverformungsrest nach DIN 53 527 bei 50%iger Stauchung ist kleiner als 45%, vorzugsweise kleiner als 30% und insbesondere kleiner als 10%;
- Dynamische Steifigkeit nach DIN 18 165 bei einer Plattendicke von 50 mm ist kleiner als 20, vorzugsweise kleiner als 10 und insbesondere kleiner als 5 [N.cm⁻³];
- Entflammbarkeit nach DIN 4102: mindestens normalentflammbar, vorzugsweise schwerentflammbar
- Zugfestigkeit im nassen Zustand > 60 J/ m²
- Oberfläche des Schaums nach der BET-Methode > 0,5 m²/g.

Die Schaumstoffe auf Basis von Melamin/Formaldehyd-Kondensationsprodukten, die erfindungsgemäß eingesetzt werden, sind offenporig. Bei einer mikroskopischen Betrachtung der Schaumstoffe zeigt es sich, daß das Schaumgerüst eine Vielzahl miteinander verbundener, dreidimensional verzweigter Stege enthält. Melamin/Formaldehyd-Harzschäume sind beispielsweise dann ausreichend elastisch, wenn die Stege die in der EP-A- 0 017 672 beschriebenen Bedingungen erfüllen, d.h. das mittlere Verhältnis von Länge zu Dicke der Stege ist größer als 10 : 1, vorzugsweise größer als 12 : 1 und insbesondere größer als 15 : 1 und die Dichte der Stege mehr als 1,10, vorzugsweise mehr als 1,20 und insbesondere mehr als 1,30 g/cm³ beträgt. Länge und Dicke der Stege werden z.B. mikroskopisch bestimmt, die Dichte der Schaumstoffstege wird durch Tauchen der Schaumstoffe in eine geeignete Flüssigkeit wie Isopropanol nach dem archimedischen Prinzip bestimmt, vgl. EP-A-0 017 672.

Gemäß dem Verfahrensschritt (b) wird der Schaumstoff während einer Zeit von 1 bis 180 Minuten bei 120 bis 300°C getempert. Er wird dabei vorzugsweise 3 bis 60 Minuten lang auf Temperaturen von 120 und 260 °C, besonders bevorzugt von 150 bis 250 °C erhitzt, wobei Wasser, Treibmittel und Formaldehyd weitgehend entfernt werden und eine Nachhärtung des Schaumharzes erfolgt. Diese Temperaturbehandlung kann unmittelbar anschließend an die Schaumherstellung in derselben Apparatur oder in einer nachgeschalteten Apparatur erfolgen; sie kann aber auch zu einem späteren Zeitpunkt unabhängig vom Schäumprozeß durchgeführt werden. Getemperte Schaumstoffe zeigen eine wesentlich geringere Neigung zum Schwinden und weisen eine geringere Gleichgewichtsfeuchte auf als ungetemperte Produkte. Auch die Formaldehyd-Emission der getemperten Schaumstoffe ist gegenüber der Formaldehydemission der ungetemperten Produkte stark verringert. Die Formaldehydabspaltung beträgt weniger als 100 mg Formaldehyd / kg Schaumstoff, bevorzugt weniger als 20 mg Formaldehyd / kg Schaumstoff (gemessen nach der EU Norm ISO 14184-1).

Die Schaumstoffe können als Platten, Blöcke oder Bahnen mit einer Höhe von bis zu 2 m hergestellt werden oder als Schaumfolien mit einer Dicke von wenigen mm, z.B. 0,5 bis 7mm. Die bevorzugte Schaumhöhe (in Schäumrichtung) liegt bei Verwendung von Mikrowellen der Frequenz 2,45 GHz zwischen 10 cm und 100 cm. Aus derartigen Schaumstoffblöcken können alle erwünschten Platten- bzw. Vliesstärken herausgeschnitten werden.

Um die Aufnahmegeschwindigkeit der Schaumstoffe für Wasser und Körperflüssigkeiten zu erhöhen, werden sie im Verfahrensschritt (c) hydrophiliert, wobei die Hydrophilierung auch bereits während des Temperns durchgeführt werden kann, indem man beispielsweise den Melamin/Formaldehydharzschaumstoff zur Entfernung aller flüchtigen Bestandteile mit heißer Luft durchströmt und dieser Temperluft hydrophilierende Substanzen beispielsweise in Form eines Aerosols hinzufügt. Auf diese Weise läßt sich eine Hydrophilierung erzielen, ohne die Schaumstoffe nachträglich mit einem Hydrophilierungsmittel behandeln zu müssen.

Die Erzeugung der notwendigen Hydrophilie im Verfahrensschritt (c) kann auf unterschiedlichen Wegen erfolgen, z.B. durch Behandlung mit mindestens einem Hydrophilierungsmittel und/oder Ozon, einer Coronaentladung oder einem Plasma.

Bei der Behandlung des Schaumstoffs mit einem Hydrophilierungsmittel kann es sich beispielsweise um eine Adsorption einer hydrophileren Komponente z.B. von Tensiden oder hydrophilen Polymeren, die gegebenenfalls eine hydrophobe Modifikation aufweisen, oder um eine chemische Anbindung von hydrophilen Reagenzien z.B. von Polyaminen, Polyepoxiden oder Polycarbonsäuren auf der Oberfläche des Schaumstoffs handeln. Eine Hydrophilierung der Oberfläche des Schaumstoffs kann auch durch Aufbringen von vernetzten Polymerisaten oder einer vernetzten, hydrophilen Hülle erfolgen, indem man z.B.
- Reagenzien, die mit sich selbst ein Netzwerk bilden können wie Kondensationsprodukte aus Epichlorhydrin und Polyamidoaminen oder Polyaminen oder
- Monomere oder Polymere, die mit einem zugesetzten Vernetzer reagieren können, z.B. Polycarbonsäuren in Kombination mit multifunktionellen Epoxiden, mehrwertigen Alkoholen oder Polyaminen, Polyamine in Kombination mit multifunktionellen Epoxiden, Acrylaten oder Estern
auf die Schaumstoffe einwirken läßt. Die Hydrophilierungsmittel werden normalerweise in gelöster Form angewandt, indem sie in einem Lösemittel gelöst werden. Sie können auch in Form von wäßrigen Dispersionen oder Dispersionen in einem organischen Lösemittel auf die zu hydrophilierenden Schaumstoffe appliziert werden. Die Hydrophilierung kann z.B. durch Eintauchen des Melamin/Formaldehyd-Schaumstoffkörpers in die Flüssigkeit erfolgen, die das Hydrophilierungsmittel in gelöster oder in dispergierter Form enthält. Alternativ kann die Flüssigkeit.mit dem gelösten oder dem dispergierten Hydrophilierungsmittel auch durch Aufsprühen auf die Schaumstoffoberfläche appliziert werden. Danach wird das Lösemittel aus dem hydrophilierten Schaumstoffkörper entfernt, z.B. durch Trocknen des Schaumstoffs.

Das Hydrophilierungsmittel reagiert mit dem zu hydrophilierenden Melamin/Formaldehydharzschaum bzw. wird an den Polymeroberflächen adsorbiert. Die Menge an zugesetztem Hydrophilierungsmittel wird so bemessen, daß einerseits eine Hydrophilierung erfolgt, ohne daß andererseits die mechanischen Eigenschaften des Schaumstoffes (Flexibilität) gestört werden. Bevorzugt wird das Hydrophilierungsmittel in einer solchen Menge zugesetzt, daß die resultierende Menge an Hydrophilierungsmittel 0,05 bis 100 Gew.-%, vorzugsweise 0,1 bis 50 Gew.-% und insbesondere 0,2 bis 30 Gew,-%, bezogen auf den Schaumstoff, beträgt.

Eine Hydrophilierung des Melamin/Formaldehydschaumstoffs ist beispielsweise durch Einwirkung mindestens eines Tensids auf den Schaumstoff möglich. Besonders bevorzugt werden hautfreundliche Tenside zugesetzt. Beispiele für hautfreundliche Hydrophilierungsmittel sind öllösliche Tenside wie Sorbitanfettsäureester, Polyglycerol-Fettsäureester und Polyoxyethylen. Beispiele für Tenside obigen Typus sind TRIODAN® 20, ein kommerziell verfügbarer Polyglycerinester, und EMSORB® 2502, ein Sorbitansesquioleat. Bevorzugte Sorbitanfettsäureester sind Sorbitanlaurat (z. B. SPAN® 20), Sorbitanmonooleat (SPAN® 80) und Kombinationen von Sorbitantrioleat (SPAN® 85) und Sorbitanmonooleat (SPAN® 80). Besonders bevorzugt ist die Kombination von Sorbitanmonooleat und Sorbitantrioleat im Gewichtsverhältnis von größer als oder gleich 3:1, insbesondere bevorzugt von 4:1. Des weiteren werden Kombinationen von Sorbitanlaurat mit bestimmten Polyglycerin-Fettsäureestern als Hydrophilierungsmittel eingesetzt. Polyglycerin-Fettsäureester werden erhalten aus esterbildenden Polyglycerihen und Fettsäuren, vgl. beispielsweise US-A-3,637,774. Die Polyglycerine werden charakterisiert durch einen hohen Anteil an linearen (v.a. acyclischen) Diglycerinen, einem geringen Anteil Tri- oder höherer Polyglycerinen, und einem geringen Anteil zyklischer Diglycerine. Das Gewichtsverhältnis von Sorbitanlaurat zu Polyglycerin-Fettsäureester liegt normalerweise zwischen 10:1 und 1:10, bevorzugt zwischen 4:1 und 1:1.

Weiterhin bevorzugt sind organisch modifizierte Polydimethylsiloxane vom Typ Nuwet® 500 oder modifizierte Silikone vom Typ Nuwet® 300 (Firma OSi). Sowohl im Falle der Polydimethylsiloxane als auch der Polysilikone sind die Produkte hydrophil modifiziert. Diese Modifizierung kann durch den Einbau von Amino, -Carboxy- oder Hydroxygruppen erfolgen. Ebenfalls möglich ist die Anbindung von Oligo- oder Polyethylenglycolseitenketten.

Als Hydrophilierungsmittel eignen sich außerdem acylierte Polyamine, die z.B. durch Reaktion von Polyaminen mit einbasischen Carbonsäuren erhältlich sind. Geeignete Polyamine sind beispielsweise Polyalkylenpolyamine mit mittleren Molmassen von 300 bis 1 Million, vorzugsweise von 500 bis 500 000. Bevorzugt eingesetzte Polyalkylenpolyamine sind Polyethylenimine.

Die einbasischen Carbonsäuren haben meistens 1 bis 18 C-Atome, z.B. Ameisensäure, Essigsäure, Propionsäure, Laurinsäure, Palmitinsäure oder Stearinsäure. In manchen Fällen ist es vorteilhaft, Mischungen aus einer langkettigen Monocarbonsäure nacheinander oder zusammen mit einem Polyalkylenpolyamin umzusetzen. Anstelle der Carbonsäuren kann man auch die Ester der Carbonsäuren einsetzen. Bei der Umsetzung der Polyalkylenpolyamine mit den Carbonsäuren oder deren Estern werden die NH₂- bzw. der NH-Gruppen der Polyalkylenpolyamine amidiert. Auf diese Weise können z.B. 5 bis 100, vorzugsweise 15 bis 85 % der Stickstoffatome im Polyalkylenpolyamin acyliert werden.

Weitere geeignete Hydrophilierungsmittel sind Polymere, die
i) wenigstens ein Polyisocyanat und
ii) wenigstens eine Verbindung mit mindestens zwei gegenüber Isocyanatgruppen reaktiven Gruppen und zusätzlich mindestens einer tertiären Aminogruppe
eingebaut enthalten. Dabei liegt im Polymer wenigstens ein Teil der tertiären Aminogruppen der Komponente ii) in Form von Ammoniumgruppen vor. Geladene kationische Gruppen lassen sich aus den tertiären Aminstickstoffen der Verbindungen der Komponente ii) und/oder des Polymers entweder durch Protonierung oder durch Quaternierung erzeugen. Im Polymer liegt dann wenigstens ein Teil der tertiären Aminogruppen in Form seiner Umsetzungsprodukte mit wenigstens einem Neutralisierungs-(Protonierungs-) und/oder Quaternierungsmittel vor.

Die Polyisocyanate i) sind vorzugsweise ausgewählt unter Verbindungen mit 2 bis 5 Isocyanatgruppen, Isocyanatpräpolymeren mit einer mittleren Anzahl von 2 bis 5 Isocyanatgruppen und Mischungen davon. Geeignet sind weiterhin Verbindungen, die zusätzlich zu oder anstelle von freien Isocyanatgruppen funktionelle Gruppen aufweisen, welche Isocyanatgruppen freisetzen oder wie Isocyanatgruppen reagieren. Dazu zählen z. B. verkappte Isocyanatgruppen, Uretdiongruppen, Isocyanuratgruppen und/oder Biuretgruppen aufweisende Verbindungen. Bei den Isocyanuratgruppen aufweisenden Verbindungen handelt es sich insbesondere um einfache Triisocyanatoisocyanurate, d. h. cyclische Trimere von Diisocyanaten, oder um Gemische mit ihren höheren, mehr als einen Isocyanuratring aufweisenden Homologen. Als Verbindungen der Komponente ii) eignen sich z.B. tertiäre Amine, bei denen der Aminstickstoff drei Substituenten aufweist, die vorzugsweise Hydroxyalkyl- und/oder Aminoalkylgruppen sind. Bevorzugt eingesetzte Verbindungen der Komponente ii) sind beispielsweise Bis(aminopropyl)methylamin, Bis(aminopropyl)piperazin, Methyldiethanolamin und Mischungen dieser Verbindungen.

Als kationische Polymere können sämtliche kationische synthetische Polymeren verwendet werden, die Amino- und/oder Ammoniumgruppen enthalten. Beispiele für solche kationische Polymere sind Vinylamineinheiten enthaltende Polymere, Vinylimidazoleinheiten enthaltende Polymere, quaternäre Vinylimidazoleinheiten enthaltende Polymere, Kondensate aus Imidazol und Epichlorhydrin, vernetzte Polyamidoamine, mit Ethylenimin gepfropfte vernetzte Polyamidoamine, Polyethylenimine, alkoxylierte Polyethylenimine, vernetzte Polyethylenimine, amidierte Polyethylenimine, alkylierte Polyethylenimine, Polyamine, Amin-Epichlorhydrin-Polykondensate, wasserlösliche Polyadditionsprodukte aus multifunktionellen Aminen mit multifunktionellen Epoxiden, alkoxylierte Polyamine, Polyallylamine, Polydimethyldiallylammoniumchloride, basische (Meth)acrylamid- oder -estereinheiten enthaltende Polymere, basische quaternäre (Meth)acrylamid- oder -estereinheiten enthaltende Polymere, und/oder Lysinkondensate.

Unter kationischen Polymeren werden auch amphotere Polymerisate verstanden, die eine netto-kationische Ladung aufweisen, d.h. die Polymeren enthalten sowohl anionische als auch kationische Monomere einpolymerisiert, jedoch ist der molare Anteil der im Polymeren enthaltenen kationischen Einheiten größer als der der anionischen Einheiten.

Zur Herstellung von Vinylamineinheiten enthaltenden Polymerisaten geht man beispielsweise von offenkettigen N-Vinylcarbonsäureamiden der Formel 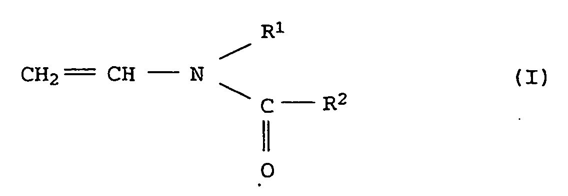 aus, in der R¹ und R² gleich oder verschieden sein können und für Wasserstoff und C₁- bis C₆-Alkyl stehen. Geeignete Monomere sind beispielsweise N-Vinylformamid (R¹=R²=H in Formel I) N-Vinyl-N-methylformamid, N-Vinylacetamid, N-Vinyl-N-methylacetamid, N-Vinyl-N-ethylacetamid, N-Vinyl-N-methylpropionamid und N-Vinylpropionamid. Zur Herstellung der Polymerisate können die genannten Monomeren entweder allein, in Mischung untereinander oder zusammen mit anderen monoethylenisch ungesättigten Monomeren polymerisiert werden. Vorzugsweise geht man von Homo- oder Copolymerisaten des N-Vinylformamids aus. Vinylamineinheiten enthaltende Polymerisate sind beispielsweise aus US-A-4 421 602, US-A-5 334 287, EP-A-02 16 387 und EP-A-0 251 182 bekannt. Sie werden durch Hydrolyse von Polymerisaten, die die Monomeren der Formel I einpolymerisiert enthalten, mit Säuren, Basen oder Enzymen erhalten.

Als monoethylenisch ungesättigte Monomere, die mit den N-Vinylcarbonsäureamiden copolymerisiert werden, kommen alle damit copolymerisierbaren Verbindungen in Betracht. Beispiele hierfür sind Vinylester von gesättigten Carbonsäuren von 1 bis 6 Kohlenstoffatomen wie Vinylformiat, Vinylacetat, Vinylpropionat und Vinylbutyrat und Vinylether wie C₁- bis C₆-Alkylvinylether, z.B. Methyl- oder Ethylvinylether. Weitere geeignete Comonomere sind ethylenisch ungesättigte C₃- bis C₆-Carbonsäuren, beispielsweise Acrylsäure, Methacrylsäure, Maleinsäure, Crotonsäure, Itaconsäure und Vinylestersäure sowie deren Alkalimetall- und Erdalkalimetallsalze, Ester, Amide und Nitrile der genannten Carbonsäuren, beispielsweise Methylacrylat, Methylmethacrylat, Ethylacrylat und Ethylmethacrylat.

Weitere geeignete Carbonsäureester leiten sich von Glykolen oder bzw. Polyalkylenglykolen ab, wobei jeweils nur eine OH-Gruppe verestert ist, z.B. Hydroxyethylacrylat, Hydroxyethylmethacrylat, Hydroxypropylacrylat, Hydroxybutylacrylat, Hydroxypropylmethacrylat, Hydroxybutylmethacrylat sowie Acrylsäuremonoester von Polyalkylenglykolen einer Molmasse von 500 bis 10000. Weitere geeignete Comonomere sind Ester von ethylenisch ungesättigten Carbonsäuren mit Aminoalkoholen wie beispielsweise Dimethylaminoethylacrylat, Dimethylaminoethylmethacrylat, Diethylaminoethylacrylat, Diethylaminoethylmethacrylat, Dimethylaminopropylacrylät, Dimethylaminopropylmethacrylat, Diethylaminopröpylacrylat, Dimethylaminobutylacrylat und Diethylaminobutylacrylat. Die basischen Acrylate können in Form der freien Basen, der Salze mit Mineralsäuren wie Salzsäure, Schwefelsäure oder Salpetersäure, der Salze mit organischen Säuren wie Ameisensäure, Essigsäure, Propionsäure oder der Sulfonsäuren oder in quaternierter Form eingesetzt werden. Geeignete Quaternierungsmittel sind beispielsweise Dimethylsulfat, Diethylsulfat, Methylchlorid, Ethylchlorid oder Benzylchlorid.

Weitere geeignete Comonomere sind Amide ethylenisch ungesättigter Carbonsäuren wie Acrylamid, Methacrylamid sowie N-Alkylmono- und Diamide von monoethylenisch ungesättigten Carbonsäuren mit Alkylresten von 1 bis 6 C-Atomen, z.B. N-Methylacrylamid, N,N-Dimethylacrylamid, N-Methylmethacrylamid, N-Ethylacrylamid, N-Propylacrylamid und tert. Butylacrylamid sowie basische (Meth)acrylamide, wie z.B. Dimethylaminoethylacrylamid, Dimethylaminoethylmethacrylamid, Diethylaminoethylacrylamid, Diethylaminoethylmethacrylamid, Dimethylaminopropylacrylamid, Diethylaminopropylacrylamid, Dimethylaminopropylmethacrylamid und Diethylaminopropylmethacrylamid.

Weiterhin sind als Comonomere geeignet N-Vinylpyrrolidon, N-Vinylcaprolactam, Acrylnitril, Methacrylnitril, N-Vinylimidazol sowie substituierte N-Vinylimidazole wie z.B. N-Vinyl-2-methylimidazol, N-Vinyl-4-methylimidazol, N-Vinyl-5-methylimidazol, N-Vinyl-2-ethylimidazol und N-Vinylimidazoline wie N-Vinylimidazolin, N-Vinyl-2-methylimidazolin und N-Vinyl-2-ethylimidazolin. N-Vinylimidazole und N-Vinylimidazoline werden außer in Form der freien Basen auch in mit Mineralsäuren oder organischen Säuren neutralisierter oder in quaternisierter Form eingesetzt, wobei die Quaternisierung vorzugsweise mit Dimethylsulfat, Diethylsulfat, Methylchlorid oder Benzylchlorid vorgenommen wird. In Frage kommen auch Diallyldialkylammoniumhalogenide wie z.B. Diallyldimethylammoniumchloride.

Außerdem kommen als Comonomere Sulfogruppen enthaltende Monomere wie beispielsweise Vinylsülfonsäure, Allylsulfonsäure, Methallylsulfonsäure, Styrolsulfonsäure, die Alkalimetall- oder Ammoniumsalze dieser Säuren oder Acrylsäure-3-sulfopropylester in Frage, wobei der Gehalt der amphoteren Copolymerisate an kationischen Einheiten den Gehalt an anionischen Einheiten übertrifft, so daß die Polymeren insgesamt eine kationische Ladung haben.

Die Copolymerisate enthalten beispielsweise
- 99,99 bis 1 mol-%, vorzugsweise 99,9 bis 5 mol-% N-Vinylcarbonsäureamide der Formel I und
- 0,01 bis 99 mol-%, vorzugsweise 0,1 bis 95 mol-% andere, damit copolymerisierbare monoethylenisch ungesättigte Monomere
in einpolymerisierter Form.

Um Vinylamineinheiten enthaltende Polymerisate herzustellen, geht man vorzugsweise von Homopolymerisaten des N-Vinylformamids oder von Copolymerisaten aus, die durch Copolymerisieren von
- N-Vinylformamid mit
- Vinylformiat, Vinylacetat, Vinylpropionat, Acrylnitril, N-Vinylcaprolactam, N-Vinylharnstoff, Acrylsäure, N-Vinylpyrrolidon oder C₁- bis C₆-Alkylvinylethern
und anschließende Hydrolyse der Homo- oder der Copolymerisate unter Bildung von Vinylamineinheiten aus den einpolymerisierten N-Vinylformamideinheiten erhältlich sind, wobei der Hydrolysegrad z. B. 0,1 bis 100 mol-% beträgt.

Die Hydrolyse der oben beschriebenen Polymerisate erfolgt nach bekannten Verfahren durch Einwirkung von Säuren, Basen oder Enzymen. Hierbei entstehen aus den einpolymerisierten Monomeren der oben angegebenen Formel I durch Abspaltung der Gruppierung 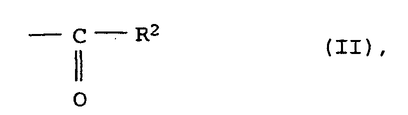 wobei R² die dafür in Formel I angegebene Bedeutung hat, Polymerisate, die Vinylamineinheiten der Formel 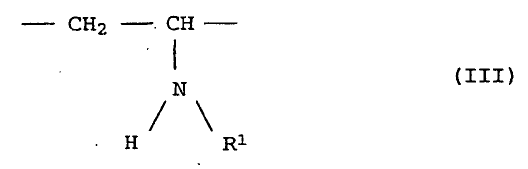 enthalten, in der R¹ die in Formel I angegebene Bedeutung hat. Bei Verwendung von Säuren als Hydrolysemittel liegen die Einheiten III als Ammoniumsalz vor.

Die Homopolymerisate der N-Vinylcarbonsäureamide der Formel, I und ihre Copolymerisate können zu 0,1 bis 100, vorzugsweise 70 bis 100 mol-% hydrolysiert sein. In den meisten Fällen beträgt der Hydrolysegrad der Homo- und Copolymerisate 5 bis 95 mol-%. Der Hydrolysegrad der Homopolymerisate ist gleichbedeutend mit dem Gehalt der Polymerisate an Vinylamineinheiten. Bei Copolymerisaten, die Vinylester einpolymerisiert enthalten, kann neben der Hydrolyse der N-Vinylformamideinheiten eine Hydrolyse der Estergruppen unter Bildung von Vinylalkoholeinheiten eintreten. Dies ist insbesondere dann der Fall, wenn man die Hydrolyse der Copolymerisate in Gegenwart von Natronlauge durchführt. Einpolymerisiertes Acrylnitril wird ebenfalls bei der Hydrolyse chemisch verändert. Hierbei entstehen beispielsweise Amidgruppen oder Carboxylgruppen. Die Vinylamineinheiten enthaltenden Homo- und Copolymeren können gegebenenfalls bis zu 20 mol-% an Amidineinheiten enthalten, die z.B. durch Reaktion von Ameisensäure mit zwei benachbarten Aminogruppen oder durch intramolekulare Reaktion einer Aminogruppe mit einer benachbarten Amidgruppe z.B. von einpolymerisiertem N-Vinylformamid entsteht. Die Molmassen der Vinylamineinheiten enthaltenden Polymerisate betragen z.B. 1000 bis 10 Millionen, vorzugsweise 10 000 bis 5 Millionen (bestimmt durch Lichtstreuung). Dieser Molmassenbereich entspricht beispielsweise K-Werten von 5 bis 300, vorzugsweise 10 bis 250 (bestimmt nach H. Fikentscher in 5 %iger wäßriger Kochsalzlösung bei 25°C und einer Polymerkonzentration von 0,5 Gew.-%.

Die Vinylamineinheiten enthaltenden Polymeren werden vorzugsweise in salzfreier Form eingesetzt. Salzfreie wäßrige Lösungen von Vinylamineinheiten enthaltenden Polymerisaten können beispielsweise aus den oben beschriebenen salzhaltigen Polymerlösungen mit Hilfe einer Ultrafiltration an geeigneten Membranen bei Trenngrenzen von beispielsweise 1000 bis 500 000 Dalton, vorzugsweise 10 000 bis 300 000 Dalton hergestellt werden. Auch die unten beschriebenen wäßrigen Lösungen von Amino- und/oder Ammoniumgruppen enthaltenden anderen Polymeren können mit Hilfe einer Ultrafiltration in salzfreier Form gewonnen werden.

Polyethylenimine werden beispielsweise durch Polymerisation von Ethylenimin in wäßriger Lösung in Gegenwart von säureabspaltenden Verbindungen, Säuren oder Lewis-Säuren hergestellt. Polyethylenimine haben beispielsweise Molmassen bis zu 2 Millionen, vorzugsweise von 200 bis 500 000. Besonders bevorzugt werden Polyethylenimine mit Molmassen von 500 bis 100 000 eingesetzt. Außerdem eignen sich wasserlösliche vernetzte Polyethylenimine, die durch Reaktion von Polyethyleniminen mit Vernetzern wie Epichlorhydrin oder Bischlorhydrinethern von Polyalkylenglykolen mit 2 bis 100 Ethylenoxid- und/oder Propylenoxid-Einheiten erhältlich sind. Auch amidische Polyethylenimine sind geeignet, die beispielsweise durch Amidierung von Polyethyleniminen mit C₁- bis C₂₂-Monocarbonsäuren erhältlich sind. Weitere geeignete kationische Polymere sind alkylierte Polyethylenimine und alkoxylierte Polyethylenimine. Bei der Alkoxylierung verwendet man z.B. pro NH-Einheit in Polyetehylenimin 1 bis 5 Ethylenoxid- bzw. Propylenoxideinheiten.

Geeignete Amino- und/oder Ammoniumgruppen enthaltende Polymere sind außerdem Polyamidoamine, die beispielsweise durch Kondensieren von Dicarbonsäuren mit Polyaminen erhältlich sind. Geeignete Polyamidoamine erhält man beispielsweise dadurch, daß man Dicarbonsäuren mit 4 bis 10 Kohlenstoffatomen mit Polyalkylenpolyaminen umsetzt, die 3 bis 10 basische Stickstoffatome im Molekül enthalten. Geeignete Dicarbonsäuren sind beispielsweise Bernsteinsäure, Maleinsäure, Adipinsäure, Glutarsäure, Korksäure, Sebacinsäure oder Terephthalsäure. Bei der Herstellung der Polyamidoamine kann man auch Mischungen von Dicarbonsäuren einsetzen, ebenso Mischungen aus mehreren Polyalkylenpolyaminen. Geeignete Polyalkylenpolyamine sind beispielsweise Diethylentriamin, Triethylentetramin, Tetraethylenpentamin, Dipropylentriamin, Tripropylentetramin, Dihexamethylentriamin, Aminopropylethylendiamin und Bis-aminopropylethylendiamin. Die Dicarbonsäuren und Polyalkylenpolyamine werden zur Herstellung der Polyamidoamine auf höhere Temperaturen erhitzt, z.B. auf Temperaturen in dem Bereich von 120 bis 220, vorzugsweise 130 bis 180°C. Das bei der Kondensation entstehende Wasser wird aus dem System entfernt. Bei der Kondensation kann man gegebenenfalls auch Lactone oder Lactame von Carbonsäuren mit 4 bis 8 C-Atomen einsetzen. Pro Mol einer Dicarbonsäure verwendet man beispielsweise 0,8 bis 1,4 Mol eines Polyalkylenpolyamins.

Weitere Aminogruppen enthaltende Polymere sind mit Ethylenimin gepfropfte Polyamidoamine. Sie sind aus den oben beschriebenen Polyamidoaminen durch Umsetzung mit Ethylenimin in Gegenwart von Säuren oder Lewis-Säuren wie Schwefelsäure oder Bortrifluoridetheraten bei Temperaturen von beispielsweise 80 bis 100°C erhältlich. Verbindungen dieser Art werden beispielsweise in der DE-B-24 34 816 beschrieben.

Auch die gegebenenfalls vernetzten Polyamidoamine, die gegebenenfalls noch zusätzlich vor der Vernetzung mit Ethylenimin gepfropft sind, kommen als kationische Polymere in Betracht. Die vernetzten, mit Ethylenimin gepfropften Polyamidoamine sind wasserlöslich und haben z.B. ein mittleres Molgewicht von 3000 bis 1 Million Dalton. Übliche Vernetzer sind z.B. Epichlorhydrin oder Bischlorhydrinether von Alkylenglykolen und Polyalkylenglykolen.

Weitere Beispiele für kationische Polymere, die Amino- und/oder Ammoniumgruppen enthalten, sind Polydiallyldimethylammoniumchloride. Polymerisate dieser Art sind ebenfalls bekannt.

Weitere geeignete kationische Polymere sind Copolymerisate aus beispielsweise 1 bis 99 mol-%, vorzugsweise 30 bis 70 mol-% Acrylamid und/oder Methacrylamid und 99 bis 1 mol-%, vorzugsweise 70 bis 30 mol-% an kationischen.Monomeren wie Dialkylaminoalkylacrylamid, -ester und/oder -methacrylamid und/oder -methacrylester. Die basischen Acrylamide und Methacrylamide liegen ebenfalls vorzugsweise in mit Säuren neutralisierter oder in quaternisierter Form vor. Als Beispiele seien genannt N-Trimethylammoniumethylacrylamidchlorid, N-Trimethylammoniumethylmethacrylamidchlorid, N-Trimethylammoniumethylmethacrylesterchlorid, N-Trimethylammoniumethylacrylesterchlorid, Trimethylammoniumethylacrylamidmethosulfat, Trimethylammoniumethylmethacrylamidmethosulfat, N-Ethyldimethylammoniumethylacrylamidethosulfat, N-Ethyldimethylammoniumethylmethacrylamidethosulfat, Trimethylammoniumpropylacrylamidchlorid, Trimethylammoniumpropylmethacrylamidchlorid, Trimethylammoniumpropylacrylamidmethosulfat, Trimethylammoniumpropylmethacrylamidmethosulfat und N-Ethyldimethylammoniumpropylacrylamidethosulfat. Bevorzugt ist Trimethylammoniumpropylmethacrylamidchlorid.

Weitere geeignete kationische Monomere für die Herstellung von (Meth)acrylamid-Polymerisaten sind Diallyldimethylammoniumhalogenide sowie basische (Meth)acrylate. Geeignet sind z.B. Copolymerisate aus 1 bis 99 mol-%, vorzugsweise 30 bis 70 mol-% Acrylamid und/oder Methacrylamid und 99 bis 1 mol-%, vorzugsweise 70 bis 30 mol-% Dialkylaminoalkylacrylaten und/oder -methacrylaten wie Copolymerisate aus Acrylamid und N,N-Dimethylaminoethylacrylat oder Copolymerisate aus Acrylamid und Dimethylaminopropylacrylat. Basische Acrylate oder Methacrylate liegen vorzugsweise in mit Säuren neutralisierter oder in quaternisierter Form vor. Die Quaternisierung kann beispielsweise mit Methylchlorid oder mit Dimethylsulfat erfolgen.

Als kationische Polymere, die Amino- und/oder Ammoniumgruppen aufweisen, kommen auch Polyallylamine in Betracht. Polymerisate dieser Art werden erhalten durch Homopolymerisation von Allylamin, vorzugsweise in mit Säuren neutralisierter oder in quaternisierter Form oder durch Copolymerisieren von Allylamin mit anderen monoethylenisch ungesättigten Monomeren, die oben als Comonomere für N-Vinylcarbonsäureamide beschrieben sind.

Die kationischen Polymerisate haben z.B. K-Werte von 8 bis 300, vorzugsweise 15 bis 180 (bestimmt nach H. Fikentscher in 5 %iger wäßriger Kochsalzlösung bei 25 % und einer Polymerkonzentration von 0,5 Gew.-%). Bei einem pH-Wert von 4,5 haben sie beispielsweise eine Ladungsdichte von mindestens 1, vorzugsweise mindestens 4 mVal/g Polyelektrolyt.

Bevorzugt in Betracht kommende kationische Polymere sind Polydimethyldiallylammoniumchlorid, Polyethylenimin, Vinylamineinheiten enthaltende Polymere, basische Monomere einpolymerisiert enthaltende Copolymere von Acrylamid oder Methacrylamid, Lysineinheiten enthaltende Polymere oder deren Mischungen. Beispiele für bevorzugt in Betracht kommende kationische Polymere sind:
Polylysine mit M_{w} von 250 bis 250 000, vorzugsweise 500 bis 100.000 sowie Lysin-Cokondensate mit Molmassen M_{w} von 250 bis 250 000, wobei man als cokondensierbare Komponente z.B. Amine, Polyamine, Ketendimere, Lactame, Alkohole, alkoxylierte Amine, alkoxylierte Alkohole und/oder nichtproteinogene Aminosäuren einsetzt,
Vinylamin-Homopolymere, 1 bis 99 % hydrolysierte Polyvinylformamide, Copolymerisate aus Vinylformamid und Vinylacetat, Vinylalkohol, Vinylpyrrolidon oder Acrylamid mit Molmassen von 3.000 - 500.000,
Vinylimidazol-Homopolymere, Vinylimiazol-Copolymere mit Vinylpyrrolidon, Vinylformamid, Acrylamid oder Vinylacetat mit Molmassen von 5.000 bis 500.000 sowie deren quaternäre Derivate,
Polyethylenimine, vernetzte Polyethylenimine oder amidierte Polyethylenimine mit Molmassen von 500 bis 3.000.000,
Amin-Epichlorhydrin-Polykondensate, die als Aminkomponente Imidazol, Piperazin, C₁-C₈-Alkylamine, C₁-C₈-Dialkylamine und/oder Dimethylaminopropylamin enthalten und die eine Molmasse von 500 bis 250.000 aufweisen, und
basische (Meth)acrylamid- oder -estereinheiten enthaltende Polymere, basische quaternäre (Meth)acrylamid- oder -estereinheiten enthaltende Polymere mit Molmassen von 10.000 bis 2.000.000.

Aminogruppen enthaltende Polymere, die als Hydrophilierungsmittel auf die Melamin/Formaldehydharz-Schaumstoffe aufgetragen sind, können gegebenenfalls darauf vernetzt werden. Eine Vernetzung der mit Aminogruppen enthaltenden Polymeren behandelten Schaumstoffe erzielt man beispielsweise durch Reaktion mit mindestens bifunktionellen Vernetzern wie Epichlorhydrin, Bischlorhydrinethern von Polyalkylenglykolen, Polyepoxiden, multifunktionellen Estern, multifunktionellen Säuren oder multifunktionellen Acrylaten.

Die erfindungsgemäßen Schaumstoffe auf Basis von Melamin/Formaldehydharzen werden in Hygieneartikeln zur Aufnahme, Verteilung und Immobilisierung von Körperflüssigkeiten, insbesondere von Blut, eingesetzt. Ihr hydrophiler Charakter erlaubt eine spontane Aufnahme wäßriger Körperflüssigkeiten. Durch die offenzellige Struktur wird ein schneller Transport ins Schaumstoffinnere gewährleistet. Bei den Hygieneartikeln, die die erfindungsgemäß zu verwendenden Schaumstoffe enthalten, handelt es sich im wesentlichen um Babywindeln, Inkontinenzprodukte, Damenhygieneartikel, Wundauflagen oder Wundverbände.

Die Schaumstoffe auf Basis Melamin/Formaldehydharz zum erfindungsgemäßen Einsatz auf dem Hygienesektor sind offenporig und hydrophil. Die Tropfenaufnahmegeschwindigkeit der erfindungsgemäßen Melamin/Formaldehyd-Schaumstoffe beträgt weniger als 5 Sekunden, bevorzugt weniger als 2 Sekunden, besonders bevorzugt weniger als 1 Sekunde.

Die offenzelligen, elastischen Schaumstoffe werden vorzugsweise als flächige Gebilde in Form von Schaumvliesen mit einer Dicke von 0,1 bis 10 mm, vorzugsweise 1 bis 5 mm in Hygieneprodukten wie Babywindeln, Inkontinenz- und Damenhygieneartikeln oder als Wundauflagen bzw. in Verbandmaterialien eingearbeitet. Die Dichte der Schaumstoffe beträgt beispielsweise 5 bis 200 g/l, vorzugsweise 10 bis 50 g/l. Die Schäume weisen vorzugsweise eine Stegstruktur auf, haben eine spezifische Oberfläche, ermittelt nach der BET-Methode, von mehr als 0,5 m²/g, z.B. 1 bis 7 m²/g, verfügen über eine Free Swell Capacity von mehr als 20 g/g, z.B. 80 bis 120 g/g und haben im nassen Zustand eine Zugfestigkeit von > 60 J/ m² , z.B. 100 bis 600 J/m².

Im allgemeinen liegt im Hygieneartikel eine Kombination aus flüssigkeitsundurchlässigem Backsheet, flüssigkeitsdurchlässigem Topsheet und absorbierender Zwischenschicht (absorbent core) vor. Derartige Hygieneartikel sind bekannt und beispielsweise in DE - G-92 18 991 und EP-A-0 689 818 beschrieben. Die absorbierende Zusammensetzung wird'zwischen Topsheet und Backsheet fixiert. Optional können elastische Bündchen und selbstklebende Verschlüsse im Hygieneartikel integriert werden. Ein bevorzugter Aufbau eines Hygieneartikels ist beispielsweise aus der US-A-3,860,003 bekannt.

Beim Einsatz der hydrophilen, offenzelligen, elastischen Schaumstoffe in einem Hygieneartikel bestehen beispielsweise zwei Möglichkeiten der Gestaltung der Zwischenschicht:
1. Die Melamin/Formaldehyd-Schaumstoffschicht dient ohne weitere Schichten als absorbierende Zwischenschicht. Sie ist dann gleichzeitig Akquisitions- bzw. Akquisitions-/Distributions-Schicht und Speicherschicht.
2. Die absorbierende Zwischenschicht besteht aus (a) einer Melamin/Formaldehyd-Schaumstoffschicht, die als Akquisitions- bzw. Akquisitions-/Distributions-Schicht wirkt und (b) einer Speicherschicht, die 10 - 100 Gew.-% hochquellfähiges Hydrogel enthält.

Die Speicherschicht stellt entweder eine Schicht aus einem Hydrogel oder Kompositionen dar, die hochquellfähige Hydrogele enthalten oder an denen sie fixiert sind. Jede Komposition ist geeignet, die die hochquellfähigen Hydrogele aufnehmen kann und die darüberhinaus in die absorbierende Zwischenschicht integriert werden kann. Eine Vielzahl derartiger Zusammensetzungen ist bereits bekannt und eingehend in der Literatur beschrieben. Eine Komposition zum Einbau der hochquellfähigen Hydrogele kann z. B. eine Fasermatrix sein, die aus einem Cellulosefasergemisch (air-laid web, wet laid web) oder aus synthetischen Polymerfasern (meltblown web, spunbonded web) oder aber aus einem Misch-Faserwerk aus Cellulosefasern und synthetischen Fasern besteht. Des weiteren können offenporige Schäume oder ähnliches zum Einbau hochquellfähiger Hydrogele dienen.

Alternativ kann eine derartige Komposition durch Fusion zweier Einzelschichten entstehen, wobei eine oder besser eine Vielzahl an Kammern gebildet werden, die die hochquellfähigen Hydrogele enthalten. In diesem Fall sollte mindestens eine der beiden Schichten wasserdurchlässig sein. Die zweite Schicht kann entweder wasserdurchlässig oder wasserundurchlässig sein. Als Schichtenmaterial können Tissues oder sonstige Gewebe, geschlossene oder offenporige Schäume, perforierte Filme, Elastomere oder Gewebe aus Fasermaterial zum Einsatz gelangen. Wenn die Speicherschicht aus einer Komposition von Schichten besteht, sollte das Schichtenmaterial eine Porenstruktur aufweisen, deren Porenabmessungen klein genug sind, um die hochquellfähigen Hydrogelpartikeln zurückzuhalten. Obige Beispiele zur Komposition der Speicherschicht schließen auch Laminate aus mindestens zwei Schichten mit ein, zwischen die hochquellfähige Hydrogele eingebaut und fixiert sein können.

Des weiteren kann die Speicherschicht aus einem Trägermaterial, wie z. B. einem Polymerfilm bestehen, auf dem die hochquellfähigen Hydrogelpartikel fixiert werden. Die Fixierung kann sowohl ein- als auch beidseitig vorgenommen werden. Das Trägermaterial kann wasserdurchlässig oder wasserundurchlässig sein.

In den obengenannten Kompositionen der Speicherschicht können die hochquellfähigen Hydrogele z.B. mit einem Gewichtsanteil von 10 bis 100 Gew.-%, bevorzugt von 40 bis 100 Gew.-% und insbesondere bevorzugt von 70 bis 100 Gew.-% vorliegen. Wenn die oben beschriebene Komposition der Speicherschicht eine Fasermatrix darstellt, so resultiert die absorbierende Zusammensetzung aus einer Mischung von Fasermaterialien und hochquellfähigen Hydrogelen.

Die Speicherschicht kann vielfältige Fasermaterialien enthalten, die als Fasernetzwerk oder Matrices zum Einsatz gelangen. Mit eingeschlossen von der vorliegenden Erfindung sind sowohl Fasern natürlichen Ursprungs (modifiziert oder unmodifiziert), als auch Synthesefasern.

Beispiele für Cellulosefasern schließen jene ein, die üblicherweise bei Absorptionsprodukten verwendet werden, wie Flauschzellstoff und Zellstoff vom Baumwolltyp. Die Materialien (Nadel- oder Laubhölzer), Herstellungsverfahren, wie chemischer Zellstoff, halbchemischer Zellstoff, chemothermischer mechanischer Zellstoff (CTMP) und Bleichverfahren sind nicht besonders eingeschränkt. So finden beispielsweise natürliche Cellulosefasern wie Baumwolle, Flachs, Seide, Wolle, Jute, Ethylcellulose und Celluloseacetat Anwendung.

Geeignete synthetische Fasern werden hergestellt aus Polyvinylchlorid, Polyvinylflourid, Polytetraflourethylen, Polyvinylidenchlorid, Polyacrylverbindungen wie ORLON®, Polyvinylacetat, Polyethylvinylacetat, löslicher oder unlöslicher Polyvinylalkohol. Beispiele für synthetische Fasern schließen thermoplastische Polyolefinfasern, wie Polyethylenfasern (PULPEX®), Polypropylenfasern und Polyethylen-Polypropylen-Zweikomponentenfasern, Polyesterfasern, wie Polyethylenterephthalatfasern (DACRON® oder KO-DEL®), Copolyester, Polyvinylacetat, Polyethylvinylacetat, Polyvinylchlorid, Polyvinylidenchlorid, Polyacryle, Polyamide, Copolyamide, Polystyrol und Copolymere der vorstehend genannten Polymere, sowie Zweikomponentenfasern aus Polyethylenterephthalat-Polyethylen-Isophthalat-Copolymer, Polyethylvinylacetat/Polypropylen, Polyethylen/Polyester, Polypropylen/Polyester, Copolyester/Polyester, Polyamidfasern (Nylon), Polyurethanfasern, Polystyrolfasern und Polyacrylnitrilfasern ein. Bevorzugt sind Polyolefinfasern, Polyesterfasern und deren Zweikomponentenfasern. Weiterhin bevorzugt sind in der Wärme haftende Zweikomponentenfasern aus Polyolefin vom Hülle-Kern-Typ und Seite-an-Seite-Typ wegen ihrer ausgezeichneten Formbeständigkeit nach der Flüssigkeitsabsorption.

Die genannten synthetischen Fasern werden bevorzugt in Kombination mit thermoplastischen Fasern eingesetzt. Bei der Hitzebehandlung migrieren letztere teilweise in die Matrix des vorhandenen Fasermaterials und stellen so beim Abkühlen Verbindungsstellen und erneute Versteifungselemente dar. Zusätzlich bedeutet der Zusatz thermoplastischer Fasern eine Erweiterung der vorliegenden Porenabmessungen nach erfolgter Hitzebehandlung. Auf diese Weise ist es möglich, durch kontinuierliches Zudosieren von thermoplastischen Fasern während der Bildung der Absorptionsschicht den Anteil thermoplastischer Fasern zum Deckblatt hin kontinuierlich zu steigern, wodurch ein ebenso kontinuierlicher Anstieg der Porengrößen resultiert. Thermoplastische Fasern können aus einer Vielzahl thermoplastischer Polymere gebildet werden, die einen Schmelzpunkt von weniger als 190ºC, bevorzugt zwischen 75ºC und 175ºC aufweisen. Bei diesen Temperaturen ist noch keine Schädigung der Cellulosefasern zu erwarten.

Die vorstehend beschriebenen Synthesefasern können beispielsweise eine Länge von 1 bis '200 mm und einem Durchmesser von 0,1 bis 100 Denier (Gramm pro 9 000 Meter) haben. Bevorzugte thermoplastische Fasern weisen eine Länge von 3 bis 50 mm, besonders bevorzugte eine Länge von 6 bis 12 mm auf. Der bevorzugte Durchmesser der thermoplastischen Faser liegt zwischen 1,4 und 10 Decitex, besonders bevorzugt zwischen 1,7 und 3,3 Decitex (Gramm pro 10 000 Meter). Die Fasern können verschiedene Formen haben , z.B. gewebeartige, schmale zylinderartige, geschnitten-/spaltgarnartige, stapelfaserartige oder endlosfaserartige.

Die Fasern in der erfindungsgemäßen absorbierenden Zusammensetzung können hydrophil oder hydrophob sein. Kombinationen aus beiden Faserformen sind möglich. Gemäß der Definition von Robert F. Gould in der Publikation "Kontaktwinkel, Benetzbarkeit und Adhäsion", American Chemical Society (1964) wird eine Faser als hydrophil bezeichnet, wenn der Kontaktwinkel zwischen der Flüssigkeit und der Faser (bzw. ihrer Oberfläche) kleiner aus 90º ist, oder wenn die Flüssigkeit zum spontanen Spreiten auf derselben Oberfläche tendiert. Beide Vorgänge sind in aller Regel coexistent. Umgekehrt wird eine Faser als hydrophob bezeichnet, wenn ein Kontaktwinkel von größer als 90º ausgebildet wird und kein Spreiten beobachtet wird.

Bevorzugt wird hydrophiles Fasermaterial eingesetzt. Besonders bevorzugt gelangt Fasermaterial zum Einsatz, das zur Körperseite hin schwach hydrophil und in der Region um die hochquellfähigen Hydrogele am stärksten hydrophil ist. Im Herstellungsprozeß wird durch den Einsatz von Schichten unterschiedlicher Hydrophilie ein Gradient erzeugt, der die auftreffende Flüssigkeit zum Hydrogel kanalisiert, wo letztendlich die Absorption erfolgt.

Geeignete hydrophile Fasern für den Einsatz in der erfindungsgemäßen absorbierenden Zwischenschicht sind beispielsweise Cellulosefasern, modifizierte Cellulosefasern, Rayon, Polyesterfasern wie z. B. Polyethylenterephthalat (DACRON®), und hydrophiles Nylon (HYDROFIL®). Geeignete hydrophile Fasern können auch erhalten werden durch Hydrophilierung hydrophober Fasern, wie z.B. die Behandlung thermoplastischer Fasern, erhalten aus Polyolefinen (wie z. B. Polyethylen oder Polypropylen, Polyamide, Polystyrole, Polyurethane usw.) mit Tensiden oder Silica. Aus Kostengründen und aus Gründen der Verfügbarkeit werden jedoch Cellulosefasern bevorzugt.

Die hochquellfähigen Hydrogelpartikeln werden in das beschriebene Fasermaterial eingebettet. Dies kann auf vielfältige Weise geschehen, indem man z. B. mit dem Hydrogelmaterial und den Fasern zusammen eine Absorptionsschicht in Form einer Matrix aufbaut, oder durch Einlagerung hochquellfähiger Hydrogele in Schichten aus Fasergemisch, wo sie letztendlich fixiert werden, sei es durch Haftmittel oder Laminierung der Schichten.

Die flüssigkeitsaufnehmende und -verteilende Fasermatrix kann dabei aus synthetischer Faser oder Cellulosefaser oder einem Gemisch aus synthetischer Faser und Cellulosefaser bestehen, wobei das Mischungsverhältnis von (100 bis 0) synthetische Faser : (0 bis 100) Cellulosefaser variieren kann. Die eingesetzten Cellulosefasern können zur Erhöhung der Formbeständigkeit des Hygieneartikels zusätzlich chemisch versteift sein.

Die chemische Versteifung von Cellulosefasern kann auf unterschiedlichen Wegen erreicht werden. Zum einen kann eine Faserversteifung erreicht werden durch Zusatz geeigneter Überzüge / Coatings zum Fasermaterial. Derartige Zusätze schließen beispielsweise Polyamid-Epichlorhydrin-Überzüge (Kymene® 557 H, Polyacrylamid- Überzüge (beschrieben in U.S. Patent 3,556,932 oder als Handelsprodukt der Marke Parez® 631 NC, Melamin-Formaldehyd-Überzüge und Polyethylenimin-Überzüge mit ein.

Die chemische Versteifung von Cellulosefasern kann auch durch chemische Reaktion erfolgen. So kann z. B. die Zugabe von geeigneten Vernetzersubstanzen eine Vernetzung bewirken, die innerhalb der Faser stattfindet. Geeignete Vernetzersubstanzen sind typische Substanzen, die zur Vernetzung von Monomeren eingesetzt werden. Mit eingeschlossen, jedoch nicht limitiert darauf, sind C₂-C₈ Dialdehyde, C₂-C₈ Monoaldehyde mit saurer Funktionalität, und insbesondere C₂-C₉ Polycarbonsäuren. Spezifische Substanzen aus dieser Reihe sind beispielswiese Glutaraldehyd, Glyoxal, Glyoxylsäure, Formaldehyd und Citronensäure. Diese Substanzen reagieren mit mindestens 2 Hydroxyl-Gruppen innerhalb einer einzelnen Cellulosekette oder zwischen zwei benachbarten Celluloseketten innerhalb einer einzelnen Cellulosefaser. Durch die Vernetzung erfolgt eine Verteifung der Fasern, die durch diese Behandlung eine größere Formbeständigkeit verliehen bekommen. Zusätzlich zu ihrem hydrophilen Charakter weisen diese Fasern einheitliche Kombinationen aus Versteifung und Elastizität auf.
Diese physikalische Eigenschaft ermöglicht es, die kapillare Struktur auch bei gleichzeitigem Kontakt mit Flüssigkeit und Kompressionskräften beizubehalten und ein vorzeitiges Kollabieren zu verhindern.

Chemisch vernetzte Cellulosefaseren sind bekannt, vgl. beispielsweise WO-A-91/11162. Die chemische Vernetzung bewirkt eine Versteifung des Fasermaterials, was sich letztendlich in einer verbesserten Formbeständigkeit des gesamten Hygieneartikels widerspiegelt. Die einzelnen Schichten werden durch dem Fachmann bekannte Methoden, wie z. B. Verschmelzen durch Wärmebehandlung, Zugabe von Schmelzklebern, Latexbindern usw. miteinander verbunden.

Generell wird in der erfindungsgemäßen Verwendung als oder in der absorbierende(n) Zwischenschicht ein hydrophiliertes Vlies aus einem offenzelligem, elastischen Melamin/Formaldehydharzschaumstoff mit sehr niedrigen Formaldehydemissionen verwendet. Die Abmessungen (Dicke) der absorbierenden Zwischenschicht liegt bei Einsatz mit der Funktionalität als absorbent core im allgemeinen zwischen 0,5 und 10 mm, bevorzugt zwischen 1 bis 5 mm. Bei Einsatz als Aquisitions- und Distributionsschicht in Kombination mit einer Speicherschicht liegt die Dicke zwischen 0,1 und 10 mm; bevorzugt zwischen 0,5 und 3 mm.

Das Topsheet kann auf unterschiedlichem Wege hergestellt werden, wie beispielsweise als Woven, Non-woven, versponnenes oder gekämmtes Fasergemisch. Bevorzugt wird gekämmtes Fasergemisch eingesetzt, das thermisch zum Topsheet gebunden wird. Das Flächengewicht des Topsheets beträgt bevorzugt 18 bis 25 g/m², eine Zugfestigkeit von mindestens 400 g/cm im trockenen Zustand und 55 g/cm im nassen Zustand.

Als Backsheet werden gewöhnlich flüssigkeitsundurchlässige Materialien eingesetzt, wie beispielsweise Polyolefine (z. B. Polyethylen-Backsheets), um die Kleidung des Trägers vor einem eventuellen Leakage zu schützen.

Die einzelnen Schichten, aus denen die Hygieneartikel aufgebaut sind, werden nach bekannten Methoden, wie Verschmelzen der Schichten durch Wärmebehandlung, Zugabe von Schmelzklebern, Latexbindern usw., miteinander verbunden. Die absorbierende Zwischenschicht wird zwischen Topsheet und Backsheet positioniert.

### Meßmethoden

### Tropfenaufnahmegeschwindigkeit

Auf eine ca. 5 mm dicke Schaumstoffschicht wird mit Hilfe einer Pipette ein einzelner Tropfen einer 0.9 %igen Kochsalzlösung aufgesetzt und die Zeit bestimmt bis der Tropfen in den Schaumstoff eingedrungen ist. War die Aufnahmezeit < 5 sec, so wurde der Schaumstoff als hydrophil bewertet.

### Dichte

Jede geeignete gravimetrische Methode kann zur Dichtebestimmung des Schaumstoffes herangezogen werden. Ermittelt wird die Masse an festem Schaumstoff pro Volumeneinheit Schaumstoffstruktur. Ein Verfahren zur Dichteermittlung des Schaumstoffes ist in der ASTM Methode Nr. D 3574-86, Test A, beschrieben. Diese Methode wurde ursprünglich zur Dichtebestimmung von Urethanschäumen entwickelt, kann aber auch zu diesem Zweck herangezogen werden. Danach wird bei einer vorkonditionierten Probe, wie in der Methode beschrieben, bei 22 +/- 2 °C deren Trockenmasse und Volumen ermittelt. Volumenbestimmungen größerer Probenabmessungen werden unter Normaldruck durchgeführt.

### Free swell capacity (FSC)

Bei dieser Methode wird die freie Quellbarkeit des offenzelligen elastischen Melamin/Formaldehyd-Schaumstoffs bestimmt. Zur Bestimmung der FSC wird aus dem Schaumstoff-Muster eine Probe geeigneter Größe, zum Beispiel eine Probe mit einer Fläche von ca. 1 cm x 1 cm, ausgeschnitten und ausgewogen. Diese Probe wird für 30 Minuten in einen Überschuß von 0,9 Gew.-%iger NaCl-Lösung gegeben (mindestens 0,83 l Kochsalz-Lösung / 1 g Schaumstoff). Die Schaumstoffprobe wird anschließend für 10 Minuten abtropfen gelassen, indem sie an einer Ecke aufgehangen wird, wobei ein Zusammenpressen der Probe unbedingt vermieden werden muß. Die Bestimmung der aufgenommenen Flüssigkeitsmenge geschieht durch Auswägen der Schaumstoff-Probe.

### Akquisitionszeit

Der offenzellige elastische Melamin/Formaldehyd-Schaumstoff wird in 1,5 mm bzw. 2 mm bzw. 4 mm dicke Schichten geschnitten. Eine kommerziell verfügbare Windel wird vorsichtig aufgeschnitten, das als Akquisitionsmedium dienende high-loft entnommen und statt dessen die offenzellige elastische Melamin/Formaldehyd-Schaumstoffschicht eingelegt. Die Windel wird wieder verschlossen. Die Aufgabe von synthetischer Harnersatzlösung erfolgt durch eine Kunststoffplatte mit einem Ring in der Mitte (Innendurchmesser des Ringes 6,0 cm, Höhe 4,0 cm). Die Platte wird belastet mit zusätzlichen Gewichten, so daß die Gesamtbelastung der Windel 13,6 g/cm² beträgt. Die Kunststoffplatte wird auf der Windel so plaziert, daß der Mittelpunkt der Windel gleichzeitig die Mitte des Aufgaberinges darstellt. Es werden dreimal 60 ml 0,9 Gew.-%ige Kochsalz-Lösung aufgegeben. Die Kochsalz-Lösung wird in einem Meßzylinder abgemessen und durch den Ring in der Platte in einem Schuß auf die Windel aufgegeben. Gleichzeitig mit der Aufgabe wird die Zeit gemessen, die zum kompletten Eindringen der Lösung in die Windel notwendig ist. Die gemessene Zeit wird als Akquisitionszeit 1 notiert. Danach wird die Windel mit einer Platte für 20 Minuten belastet, wobei die Belastung weiterhin bei 13,6 g/cm² gehalten wird. Danach erfolgt die zweite Aufgabe der Flüssigkeit. Die gemessene Zeit wird als Akquisitionszeit 2 notiert. Bei der Bestimmung von Akquisitionszeit 3 wird auf die gleiche Weise verfahren.

### Spezifische Oberfläche

Die Ermittlung der spezifischen Oberfläche erfolgt nach der BET-Methode gemäß DIN 66132.

### Formaldehyd-Emission

### Die Bestimmung erfolgte nach der Edana-Methode 210.1-99 (Prüfung nach EU Norm EN ISO 14184-1)

1g der zu untersuchenden Schaumprobe wird in kleine Stücke geschnitten zusammen mit 100 ml Wasser in einen Erlenmeyerkolben gefüllt und dicht verschlossen. Der Erlenmeyerkolben wird in ein auf 40°C temperiertes Wasserbad gesetzt und dort unter regelmäßigem Schütteln für 60 min belassen. Anschließend wird die erhaltene Lösung abfiltriert bzw. der Schaum ausgedrückt.
In der erhaltenen Lösung wird der Formaldehydgehalt nach dem Acetylaceton-Verfahren bestimmt.

Sofern aus dem Zusammenhang nichts anderes hervorgeht, bedeuten die Prozentangaben in den Beispielen Gewichtsprozent.

### Beispiele

### Vergleichsbeispiel 1

75 Teile eines sprühgetrockneten Melamin/Formaldehyd-Vorkondensates (Molverhältnis 1:3) wurden in 25 Teilen Wasser gelöst. Dieser Harzlösung wurden 3 % Ameisensäure, 2 % eines Na- C₁₂/C₁₈-Alkansulfonats und 19 % Pentan, jeweils bezogen auf das Harz, zugesetzt. Die Mischung wurde kräftig gerührt und anschließend in einer Schäumform aus Polypropylen durch Einstrahlung von Mikrowellenenergie bei 2,54 GHz verschäumt. Der Schaumstoff wurde bei 100ºC getrocknet und anschließend bei 220ºC 30 min getempert. Der so hergestellte Melamin/Formaldehyd.-Schaumstoff war hydrophil und hatte eine Dichte von 10 g/l. Die nach 1 h Lagerung bei 40ºC in Wasser gemessene Formaldehydfreisetzung betrug 150 mg Formaldehyd / kg Schaumstoff. Die freie Aufnahmekapazität (FSC), bestimmt durch Teebeuteltest, betrug 103 g/g. Der Schaustoff hatte eine spezifische Oberfläche, bestimmt nach BET-Methode, von 5,3 m²/g.

### Vergleichsbeispiel 2

70 Teile eines sprühgetrockneten Melamin/Formaldehyd-Vorkondensates (Molverhältnis 1:1,6) wurden in 30 Teilen Wasser gelöst. Dieser Harzlösung wurden 3 % einer Emulgatormischung aus einem Alkanolamid und einem ethoxylierten Fettalkohol sowie 3 % Ameisensäure umd 10 % Pentan zugesetzt. Die Mischung wurde verschäumt, der Schaum getrocknet und getempert wie im Vergleichsbeispiel 1 beschrieben. Der auf diese Weise hergestellte Schaumstoff war hydrophob, seine Dichte betrug ebenfalls 10 g/l. Die Formaldehydabspaltung betrug weniger als 20 mg Formaldehyd / kg Schaumstoff.

### Beispiele 1 bis 15

5 mm starke Schaumstoffschichten des nach Vergleichsbeispiel 2 hergestellten Schaumstoffs wurden in 1 %ige wäßrige Lösungen der in Tabelle 1 angegebenen Beschichtungsmittel eingelegt und durch Walken vollständig benetzt. Die Schaumproben wurden nach jeweils 30 min aus der Lösung entnommen, abgepresst und an der Luft für ca. 18 Stunden vorgetrocknet. Anschließend wurden die Proben bei 110°C im Vakuum für 1 Stunde getrocknet. Danach waren alle behandelten Schaumstoffproben hydrophiliert, vgl. Tabelle 1.

### Herstellung von Tensid 1

121 g einer 53%igen wässrigen Lösung eines Polyethylenimins mit einem zahlenmittlerem Molekulargewicht von ca. 5000 wurde bei 120°C und 25 mbar entwässert. Bei 80°C wurde das unter Stickstoff gerührte Polyethylenimin mit 15,5 g C₁₂-C₁₄-Fettsäure (SZ 271 g KOH/g) (Edenor® C 12 70, Fa. Henkel) versetzt. Nach Erwärmen wurde 6 Stunden bei 160°C gerührt. Das sich bildende Reaktionswasser wurde abdestilliert. Nach Abkühlen auf 140°C wurden 51,8 g Ameisensäure zugetropft und 4,5 Stunden bei 140°C nachgerührt. Nach beendeter Amidierung wurde auf 90°C abgekühlt und unter Rühren 230 ml vollentsalztes Wasser zugegeben. Man erhielt 332 g einer 31,5%igen wässrigen Lösung eines zu 5,0% mit C₁₂-C₁₄-Fettsäure und erschöpfend mit Ameisensäure amidierten Polyethylenimins.

### Herstellung von Tensid 2

In einem Vierhalskolben, der mit Rührer, Tropftrichter, Thermometer und Rückflusskühler ausgestattet war, wurden 20,0 g (0,1 Mol) Bis(aminopropyl)piperazin in 200 g Aceton gelöst. Dazu wurden 22,2 g (0,1 Mol) Isophorondiisocyanat so zugetropft, dass die Temperatur nicht über 30 °C stieg. Das Reaktionsgemisch wurde eine weitere Stunde am Rückfluss gerührt und anschließend 110 g HCl (1 n) und 100 g Wasser zugegeben. Anschließend wurde das Aceton unter vermindertem Druck abdestilliert. Man erhielt eine Polyharnstofflösung mit einem Feststoffgehalt von 16,7 Gew.-% und einem pH-Wert von 7,2.

Meßbedingungen der K-Werte für die in Tabelle 1 genannten Polymeren:

| Polymer | Lösemittel | Polymer-Konzentration der Lösung [Gew.-%] | pH der Lösung |
|---|---|---|---|
| Polyvinylamin | 3%ige wässrige NaCl-Lösung | 0,5 | 11,0 |
| Polyacrylsäure | Wasser | 1,0 | 7,0 (neutralisiert mit NaOH) |
| Polysin | Wasser | 1,0 | 10,3 |
| Copolymer aus Acrylamid und Vinylimidazol | 3%ige wässrige NaCl-Lösung | 0,1 | 8,0 |
| Capolymer aus Acrylamid und N-Trimethylammoniumethylacrylesterchlorid | 3%ige wässrige NaCl-Lösung | 0,1 | 4,8 ! |

**Tabelle 1**

| Beispiel | Beschichtungsmittel | Hydrophil | Hydrophob |
|---|---|---|---|
| Vergleich 1 | - | X | |
| Vergleich 2 | - | | X |
| | | | |
| 1 | Diglycerinmonooleat | X | |
| 2 | Sorbitanmonooleat | X | |
| 3 | Umsetzungsprodukt eines ungesättigten C₁₃C₁₅-Oxoalkohols mit jeweils 4 Ethylenoxideinheiten und jeweils 4 Propylenoxideinheiten | X | |
| 4 | Gemisch aus 25 % Stearylalkohol und 75 % eines Umsetzungsproduktes aus Cetylstearylalkohols mit 6 Ethylenoxideinheiten | X | |
| 5 | Polyacrylsäure, K-Wert 110 | X | |
| 6 | Polyvinylamin, K-Wert 90 | X | |
| 7 | N-Methylaminopropyltrimethoxysilan | X | |
| 8 | Amino-modifiziertes Silikon-Polyethercopolymer, Nu-wet®300, Firma OSi | X | |
| 9 | Polyalkylenoxid-modifiziertes Polydimethylsiloxan, Nu-wet 500, Firma OSi | X | |
| 10 | Organo-modifiziertes Polymethylsiloxan, Nuwet® 100, Firma OSi | X | |
| 11 | Reaktionsgemisch aus Polyvinylamin (K-Wert 90) und Ethylenglycoldiglydidylether im Gew.-Verhältnis 40/1 | X | |
| 12 | Copolymer aus Acrylamid und Vinylimidazol mit einem molaren Verhältnis von 1/1, K-Wert 30 | X | |
| 13 | Copolymer aus Acrylamid und N-Trimethylammoniumethylacrylesterchlorid mit einem molaren Verhältnis von 1/1, K-Wert 30 | X | |
| 14 | Tensid 1 | X | |
| 15 | Tensid 2 | X | |

### Beispiel 16

Der nach Beispiel 10 hydrophilierte Melamin-Formaldehydharz-Schaum wurde in 2 mm dicke Schichten geschnitten. Eine kommerziell verfügbare Windel wurde vorsichtig aufgeschnitten, das high-loft entnommen und statt dessen die 2 mm dicke Schaumschicht eingelegt. Die Windel wurde wieder verschlossen. Danach wurden die Zeiten bestimmt, die für die Aufnahme von 3 aufeinanderfolgenden Zugaben von jeweils 60 ml synthetischem Urin benötigt wurden. Die Meßwerte sind in Tabelle 2 angegeben.

### Beispiel 17

Entsprechend dem Beispiel 16 wurde eine entsprechend Beispiel 6 hydrophilierter Schaum in eine Windel eingearbeitet und die Akquisitionszeiten bestimmt. Die Ergebnisse sind in Tabelle 2 angegeben.

### Vergleichsbeispiel 3

Eine kommerziell verfügbare Windel wurde vorsichtig aufgeschnitten, das high-loft entnommen, anschließend wieder eingelegt und die Windel wiederum verschlossen. Diese Vorgehensweise sollte eine optimale Vergleichbarkeit sicherstellen. Anschließend wurden die Akquisitionszeiten bestimmt. Die Ergebnisse sind in Tabelle 2 angegeben.

### Vergleichsbeispiel 4

Der nach Vergleichsbeispiel 2 hergestellte formaldehydarme Schaum aus Melamin/Formaldehyd-Kondensat wurde wie in Beispiel 16 beschrieben in eine Windel eingearbeitet. Anschließend wurden die Akquisitionszeiten bestimmt. Die Ergebnisse sind in Tabelle 2 angegeben.

### Vergleichsbeispiel 5

Der nach Vergleichsbeispiel 1 hergestellte Schaum aus Melamin/Formaldehyd-Kondensat wurde wie in Beispiel 16 beschrieben in eine Windel eingearbeitet. Anschließend wurden die Akquisitionszeiten bestimmt. Die Ergebnisse sind in Tabelle 2 angegeben.

**Tabelle 2**

| Windel | Zeit für die Aufnahme der ersten 60 ml [sec] | Zeit für die Aufnahme der zweiten 60 ml [sec] | Zeit für die Aufnahme der dritten 60 ml [sec] |
|---|---|---|---|
| Vgl.beispiel 3 | 7 | 19 | 29 |
| Vgl.beispiel 4 | 80 | 125 | 148 |
| Vgl.beispiel 5 | 3 | 6 | 8 |
| Beispiel 16 | 3 | 4 | 6 |
| Beispiel 17 | 4 | 6 | 8 |

Die Tabelle 2 lässt erkennen, dass die Akquisitionszeiten des hydrophilierten, formaldehydarmen Schaums signifikant besser sind als die einer kommerziell erhältlichen Windel und auf dem gleichen Niveau liegen wie bei dem ursprünglichen formaldehydreichen Schaum, der nach Vergleichsbeispiel 1 hergestellt wurde.

## Patentansprüche

1. Hydrophile, offenzellige, elastische Schaumstoffe aus Melamin/Formaldehyd-Harzen, **dadurch gekennzeichnet, daß** sie eine Tropfenaufnahmegeschwindigkeit von weniger als 5 Sekunden und eine Formaldehyd-Emission nach der EU Norm EN ISO 14184-1 von weniger als 100 mg Formaldehyd / kg Schaustoff haben.

2. Hydrophile, offenzellige, elastische Schaumstoffe nach Anspruch 1, **dadurch gekennzeichnet, daß** sie eine Dichte von 5 bis 200 g/l, eine spezifische Oberfläche (bestimmt nach BET) von mehr als 0,5 m²/g und eine Free Swell Capacity von mehr als 20 g/g haben.

3. Hydrophile, offenzellige, elastische Schaumstoffe nach Anspruch 1 oder 2, **dadurch gekennzeichnet, daß** sie im nassen Zustand eine Zugfestigkeit von >60 J/m² aufweisen.

4. Verfahren zur Herstellung von hydrophilen, offenzelligen, elastischen Schaumstoffen nach einem der Ansprüche 1 bis 3, **dadurch gekennzeichnet, daß** man
(a) eine wäßrige Lösung oder Dispersion, die jeweils mindestens ein Melamin/Formaldehyd-Vorkondensat, einen Emulgator, ein Treibmittel und einen Härter enthalten, unter Ausbildung eines Schaumstoffs und Vernetzen des Vorkondensates erhitzt,
(b) den Schaumstoff dann während einer Zeit von 1 bis 180 Minuten bei 120 bis 300°C tempert, wobei flüchtige Anteile entfernt werden, und
(c) ihn während des Temperns oder danach mit mindestens einem Hydrophilierungsmittel und/oder mit Ozon, einer Coronaentladung oder einem Plasma behandelt.

5. Verfahren nach Anspruch 4, **dadurch gekennzeichnet, daß** man ein Melamin/Formaldehyd-Vorkondensat einsetzt, bei dem das Molverhältnis Melamin zu Formaldehyd 1 : 1,0 bis 1 : 1,9 beträgt.

6. Verfahren nach Anspruch 4 oder 5, **dadurch gekennzeichnet, daß** man ein Melamin/Formaldehyd-Vorkondensat einsetzt, bei dem das Molverhältnis Melamin zu Formaldehyd 1 : 1,3 bis 1 : 1,8 beträgt.

7. Verfahren nach einem der Ansprüche 4 bis 6, **dadurch gekennzeichnet, daß** man als Hydrophilierungsmittel mindestens ein Tensid einsetzt.

8. Verfahren nach einem der Ansprüche 4 bis 6, **dadurch gekennzeichnet, daß** man als Hydrophilierungsmittel Amino- und/oder Ammoniumgruppen enthaltende Polymere einsetzt.

9. Verfahren nach einem der Ansprüche 4 bis 6, **dadurch gekennzeichnet, daß** man als Hydrophilierungsmittel Polyalkylenglykole und/oder Polymerisate von monoethylenisch ungesättigten Carbonsäuren einsetzt.

10. Verfahren nach Anspruch 8, **dadurch gekennzeichnet, daß** man als Hydrophilierungsmittel Vinylamineinheiten enthaltende Polymerisate und/oder Polyethylenimine einsetzt.

11. Hydrophile, offenzellige, elastische Schaumstoffe, **dadurch gekennzeichnet, daß** sie erhältlich sind nach dem Verfahren nach einem der Ansprüche 4 bis 10.

12. Verwendung der hydrophilen, offenzelligen, elastischen Schaumstoffe aus Melamin/Formaldehyd-Harzen nach den Ansprüchen 1 bis 3 in Hygieneartikeln zur Aufnahme, Verteilung und Immobilisierung von Körperflüssigkeiten.

13. Verwendung nach Anspruch 11, **dadurch gekennzeichnet, daß** es sich bei den Hygieneartikeln um Babywindeln, Inkontinenzprodukte, Damenhygieneartikel, Wundauflagen oder Wundverbände handelt.

## Claims

1. Hydrophilic open-celled resilient foams comprising melamine-formaldehyde resins, **characterized by** a droplet absorption rate of less than 5 seconds and an EU standard EN ISO 14184-1 formaldehyde emission of less than 100 mg of formaldehyde / kg of foam.

2. Hydrophilic open-celled resilient foams as claimed in claim 1, **characterized by** a density of from 5 to 200 g/l, a specific surface area (determined according to BET) of more than 0.5 m²/g and a Free Swell Capacity of more than 20 g/g.

3. Hydrophilic open-celled resilient foams as claimed in claim 1 or 2, **characterized by** a tensile strength of >60 J/m² in the wet state.

4. A process for preparing hydrophilic open-celled resilient foams as claimed in any of claims 1 to 3, which comprises
(a) heating an aqueous solution or dispersion each containing at least a melamine-formaldehyde precondensate, an emulsifier, a blowing agent and a curing agent to form a foam and crosslink the precondensate,
(b) then conditioning the foam at from 120 to 300°C for from 1 to 180 minutes to remove volatiles, and
(c) treating the foam during the conditioning or thereafter with at least one hydrophilicizer and/or with ozone, a corona discharge or a plasma.

5. A process as claimed in claim 4, wherein the melamine-formaldehyde precondensate used has a molar ratio of melamine to formaldehyde in the range from 1 : 1.0 to 1 : 1.9.

6. A process as claimed in claim 4 or 5, wherein the melamine-formaldehyde precondensate used has a molar ratio of melamine to formaldehyde in the range from 1 : 1.3 to 1 : 1.8.

7. A process as claimed in any of claims 4 to 6, wherein the hydrophilicizer used is at least one surfactant.

8. A process as claimed in any of claims 4 to 6, wherein the hydrophilicizer used is polymers containing amino and/or ammonium groups.

9. A process as claimed in any of claims 4 to 6, wherein the hydrophilicizer used is polyalkylene glycols and/or polymers of monoethyleneically unsaturated carboxylic acids.

10. A process as claimed in claim 8, wherein the hydrophilicizer used is polymers containing vinylamine units and/or polyethyleneimines.

11. Hydrophilic open-celled resilient foams obtainable by the process of any of claims 4 to 10.

12. The use of the hydrophilic open-celled resilient foams comprising melamine-formaldehyde resins according to any of claims 1 to 3 in hygiene articles to acquire, distribute and immobilize body fluids.

13. The use of claim 12, wherein the hygiene articles are infant diapers, incontinence products, femcare articles, wound contact materials or secondary wound dressings.

## Revendications

1. Mousses élastiques, hydrophiles, à cellules ouvertes, à base de résines de mélamine/formaldéhyde, **caractérisées en ce qu'**elles présentent une vitesse d'absorption de gouttes de moins de 5 secondes et une émission de formaldéhyde selon la norme européenne EN ISO 14184-1 de moins de 100 mg de formaldéhyde/kg de mousse.

2. Mousses élastiques, hydrophiles, à cellules ouvertes, selon la revendication 1, **caractérisées en ce qu'**elles présentent une densité de 5 à 200 g/litre, une surface spécifique (déterminée selon BET) de plus de 0,5 m²/g et une Free Swell Capacity de plus de 20 g/g.

3. Mousses élastiques, hydrophiles, à cellules ouvertes, suivant la revendication 1 ou 2, **caractérisées en ce qu'**elles présentent à l'état humide une résistance à la traction de >60 J/m².

4. Procédé de préparation de mousses élastiques, hydrophiles, à cellules ouvertes, suivant l'une des revendications 1 à 3, **caractérisé en ce que**
(a) on chauffe une solution ou dispersion aqueuse qui contient respectivement au moins un produit de précondensation de mélamine/formaldéhyde, un agent émulsionnant, un agent moussant et un durcisseur, avec réalisation d'une mousse et réticulation du produit de précondensation,
(b) on recuit ensuite la mousse à 120 jusqu'à 300°C pendant une période de 1 à 180 minutes, des fractions volatiles étant éliminées, et
(c) pendant le recuit ou après celui-ci, on la traite avec au moins un agent d'hydrophilisation et/ou avec de l'ozone, une décharge corona ou un plasma.

5. Procédé suivant la revendication 4, **caractérisé en ce qu'**on met en oeuvre un produit de précondensation de mélamine/formaldéhyde dans lequel le rapport molaire entre la mélamine et le formaldéhyde est de 1/1,0 à 1/1,9.

6. Procédé suivant la revendication 4 ou 5, **caractérisé en ce qu'**on met en oeuvre un produit de précondensation de mélamine/formaldéhyde dans lequel le rapport molaire entre la mélamine et le formaldéhyde est de 1/1,3 à 1/1,8.

7. Procédé suivant l'une des revendications 4 à 6, **caractérisé en ce que**, comme agent d'hydrophilisation, on met en oeuvre au moins un agent tensioactif.

8. Procédé suivant l'une des revendications 4 à 6, **caractérisé en ce que**, comme agent d'hydrophilisation, on met en oeuvre des polymères contenant des groupes amino et/ou ammonium.

9. Procédé suivant l'une des revendications 4 à 6, **caractérisé en ce que**, comme agent d'hydrophilisation, on met en oeuvre des polyalkylèneglycols et/ou des polymères d'acides carboxyliques monoéthyléniquement insaturés.

10. Procédé suivant la revendication 8, **caractérisé en ce que**, comme agent d'hydrophilisation, on met en oeuvre des polymères contenant des unités vinylamine et/ou des polyéthylèneimines.

11. Mousses élastiques, hydrophiles, à cellules ouvertes, **caractérisées en ce qu'**elles peuvent être obtenues selon le procédé suivant l'une des revendications 4 à 10.

12. Utilisation des mousses élastiques, hydrophiles, à cellules ouvertes, à base de résines de mélamine/formaldéhyde, suivant les revendications 1 à 3, dans des articles d'hygiène pour l'absorption, la répartition et l'immobilisation de liquides corporels.

13. Utilisation suivant la revendication 11, **caractérisée en ce que**, en ce qui concerne les articles d'hygiène, il s'agit de langes pour bébé, de produits pour l'incontinence, d'articles d'hygiène féminine, de bandages ou de pansements.
